# EUROPÄISCHE PATENTSCHRIFT

(11) **EP 0 759 433 B1**
(45) Veröffentlichungstag und Bekanntmachung des Hinweises auf die Patenterteilung: **16.06.1999**
(21) Anmeldenummer: 96112760.2
(22) Anmeldetag: 08.08.1996
(51) Int. Cl.: C07D 317/46

(54) **Verfahren zur Herstellung von 2,2-Difluorbenzo[1.3]dioxolcarbaldehyden**
Process for the preparation of 2,2-difluorobenzo[1.3]dioxolcarbaldehydes
Procédé de préparation de 2,2-difluorobenzo[1.3]dioxol-carbaldéhydes

(30) Priorität: 21.08.1995 DE 19530637
(43) Veröffentlichungstag der Anmeldung: 26.02.1997
(73) Patentinhaber: BAYER AG, 51368 Leverkusen (DE)
(72) Erfinder: Andres, Peter, Dr., 42799 Leichlingen (DE); Marhold, Albrecht, Dr., 51373 Leverkusen (DE)

(56) Entgegenhaltungen:
- EP-A- 0 291 799
- CH-A- 676 119
- HOUBEN-WEYL: METHODEN DER ORGANISCHEN CHEMIE, Bd. VII/1, 1954, GEORG THIEME VERLAG STUTTGART DE, Seiten 210-217, XP002018709 "Sauerstoffverbindungen II, Teil 1: Aldehyde"

## Beschreibung

Die vorliegende Erfindung betrifft ein besonders vorteilhaftes Verfahren zur Herstellung von 2,2-Difluorbenzo[1.3]dioxolcarbaldehyden aus Methylbenzo[1.3]dioxolen.

2,2-Difluorbenzo[1.3]dioxolcarbaldehyde sind bekannte Zwischenprodukte für Pflanzenschutzmittel und pharmazeutische Wirkstoffe (vgl. EP-A 634 413, EP-A 606 843, DE-A 4 029 444, EP-A 333 658, US-A 5 344 944 und EP-A 291 799).

Für die Herstellung von 2,2-Difluorbenzo[1.3]dioxolcarbaldehyden sind bisher 3 Verfahren bekannt, die hier am Beispiel der Herstellung von 2,2-Difluorbenzo[1.3]dioxol-4-carbaldehyd erläutert werden.

Verfahren 1 (vgl. EP-A 291 799 und US-A 5 344 944): 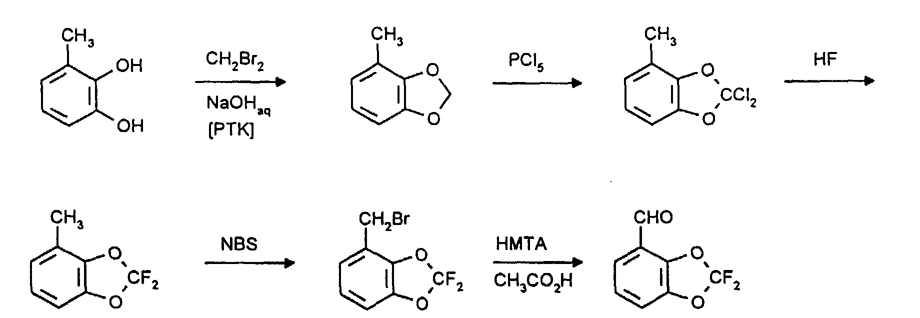 PTK bedeutet Phasentransferkatalysator, NBS bedeutet N-Bromsuccinimid, HMTA bedeutet Hexamethylentetramin (= Urotropin).

Verfahren 2 (vgl. EP-A 333 658): 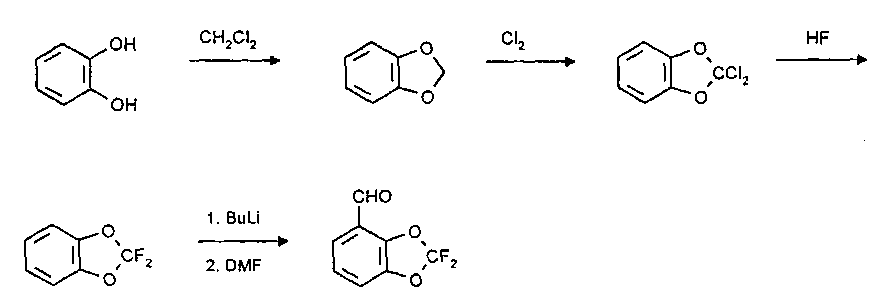 BuLi bedeutet Butyllithium, DMF bedeutet Dimethylformamid.

Gegenstand der EP-A 333 658 ist nur die letzte Verfahrensstufe. Die vorher durchzuführenden Verfahrensstufen sind der Vergleichbarkeit wegen angegeben.

Verfahren 3 (vgl. DE-A 4 133 155): 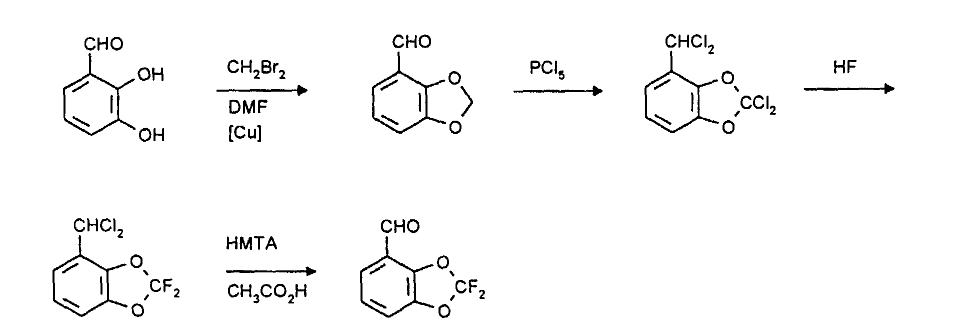 DMF bedeutet Dimethylformamid, HMTA bedeutet Hexamethylentetramin.

Gegenstand der DE-A 4 133 155 ist nur das Verfahren ab der Umsetzung von Benzo[1.3]dioxo-4-carbaldehyd mit Phosphorpentachlorid. Die vorher durchzuführende Verfahrensstufe ist der Vergleichbarkeit wegen angegeben.

Nach den Verfahren 1 und 3 kann auch 2,2-Difluorbenzo[1.3]dioxo-5-carbaldehyd hergestellt werden. Das Verfahren 2 ist auf die Herstellung des 4-Carbaldehyds beschränkt.

Nachteilig bei den bekannten Verfahren ist, daß sie 4 oder 5 Stufen aufweisen, hinsichtlich der erzielbaren Ausbeute nicht immer befriedigend sind, teilweise teure und schwierig zugängliche und handhabbare Chemikalien erfordern und größere Mengen zu entsorgender Rückstände anfallen.

Es wurde nun ein Verfahren zur Herstellung von 2,2-Difluorbenzo[1.3]dioxolcarbaldehyden der Formel (I) gefunden 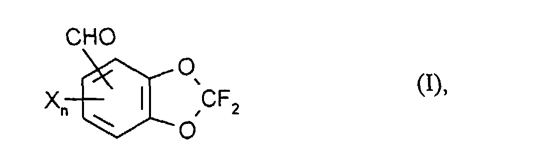 wobei
- X: für Fluor und/oder Chlor und
- n: für Null oder eine ganze Zahl von 1 bis 3 stehen,
das dadurch gekennzeichnet ist, daß man Methylbenzo[1.3]dioxole der Formel 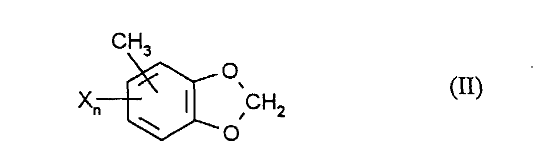 wobei X und n die bei Formel (I) angegebene Bedeutung haben,
chloriert, das erhaltene Dichlormethyl-2,2-dichlorbenzo[1.3]dioxol mit Fluorwasserstoff fluoriert und das dabei erhaltene Dichlormethyl-2,2-difluorbenzo[1.3]dioxol mit einer Carbonsäure umsetzt.

Das erfindungsgemäße Verfahren kann durch folgendes Formelschema illustriert werden:

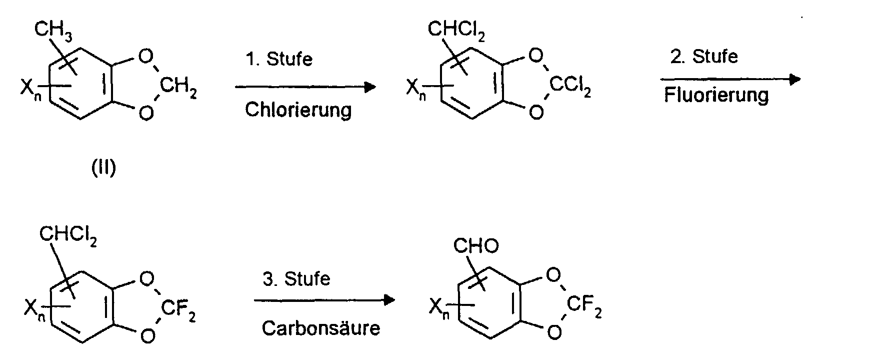

Man kann von gegebenenfalls kernfluorierten und/oder gegebenenfalls kernchlorierten 4-Methyl- oder 5-Methyl-benzo[1.3]-dioxolen ausgehen und entsprechenden 2,2-Difluorbenzo[1.3]dioxol-4- oder -5-carbaldehyde herstellen.

Für die Chlorierung in der 1. Stufe kommen als Chlorierungsmittel beispielsweise Chlor, Phosphorchloride wie PCl₃ und PCl₅, Sulfurylchlorid und Gemische davon in Frage. Bevorzugt ist Chlor. Das Chlorierungsmittel kann z.B. in Mengen von über 3,5 Chlorierungsäquivalenten pro Mol Methylbenzo[1.3]dioxol der Formel (II) eingesetzt werden. Vorzugsweise beträgt dieses Verhältnis 4:1 bis 10:1, insbesondere 4,1:1 bis 4,5:1.

Die Chlorierungstemperatur kann z.B. im Bereich 0 bis 230°C liegen. Wenn man ohne Zusatz von Radikalstartern und in Abwesenheit von UV-Licht arbeitet sind Temperaturen von 60 bis 210°C, insbesondere 80 bis 180°C, bevorzugt. Vorzugsweise arbeitet man unter Zusatz von Radikalstartern oder in Gegenwart von UV-Licht. Dann sind Temperaturen von 40 bis 180°C, insbesondere 50 bis 160°C, bevorzugt. Als Radikalstarter kommen z.B. in Frage: organische Peroxide wie Dibenzoylperoxid (DBPO) und aliphatische Azoverbindungen wie α,α'-Azoisobutyronitril (AIBN).

Radikalstarter kann man z.B. in Mengen von 0,1 bis 10 mol-% zusetzen. Es ist außerdem bevorzugt, die Chlorierung bei relativ tieferer Temperatur, z.B. bei 40 bis 100°C zu beginnen und bei relativ höherer Temperatur, z.B. bei 120 bis 180°C zu Ende zu führen. Man kann die Chlorierung in Gegenwart eines Lösungsmittels durchführen, z.B. in Gegenwart eines perchlorierten Kohlenwasserstoffs oder eines halogenierten Aromaten wie Chlorbenzol oder Chlorbenzotrifluorid. Vorzugsweise arbeitet man ohne Lösungsmittelzusatz.

Vorzugsweise führt man die Chlorierung so durch, daß man das Edukt vorlegt und das Chlorierungsmittel zudosiert.

Nach der Chlorierung liegt ein Gemisch vor, das im allgemeinen neben dem gewünschten Dichlormethyl-2,2-dichlorbenzo[1.3]dioxol in kleineren Mengen die entsprechende Methylverbindung, die entsprechende Monochlormethylverbindung und die entsprechende Trichlormethylverbindung enthält. Man kann dieses Gemisch destillativ auftrennen, vorzugsweise bei vermindertem Druck. Die unterchlorierten Bestandteile des Gemisches können in die Chlorierung zurückgeführt werden.

In die Fluorierung der 2. Stufe kann man z.B. mindestens 2 mol Fluorwasserstoff pro Mol des jeweiligen Dichlormethyl-2,2-dichlorbenzo[1.3]dioxols einsetzen. Vorzugsweise beträgt diese Menge 2 bis 40 mol, insbesondere 5 bis 30 mol. Die Temperatur kann beispielsweise im Bereich -35 bis +180°C liegen. Bevorzugt sind -25 bis +40°C, insbesondere -20 bis +15°C. Man kann die Fluorierung in Gegenwart eines Lösungsmittels durchführen, z.B. in Gegenwart von inerten aliphatischen oder aromatischen Lösungsmitteln wie Methylenchlorid und Nitrobenzol. Vorzugsweise arbeitet man ohne Lösungsmittelzusatz. Die Reihenfolge der Zugabe der Reaktionspartner ist nicht von besonderer Bedeutung. Bei Temperaturen über 0°C ist bevorzugt das Edukt vorzulegen und Fluorwasserstoff zuzudosieren.

Das nach der Fluorierung vorliegende Gemisch kann man z.B. so aufarbeiten, daß man zunächst die Hauptmenge überschüssigen Fluorwasserstoffs entfernt, z.B. durch Abziehen im Vakuum, den verbleibenden Rest auf Eiswasser schüttet und die organische Phase abtrennt, trocknet und destilliert. Man erhält so ein für die Durchführung der 3. Stufe geeignetes Dichlormethyl-2,2-difluorbenzo[1.3]dioxol.

Für die Umsetzung mit einer Carbonsäure kommen die verschiedensten Carbonsäuren in Frage. Die Carbonsäuren können gesättigt oder ungesättigt, z.B. aromatisch, sein, sie können ein- oder mehrbasisch sein und sie können gegebenenfalls Substituenten enthalten, beispielsweise einen oder mehrere Halogen-, Nitro-, Carbonyl-, Alkoxycarbonyl-, Hydroxy- und/oder Cyano-Substituenten. Bevorzugt sind geradkettige und verzweigte 1- bis 3-basische C₁-C₁₀-Alkyl- und 1- bis 3-basische C₆-C₁₂-Aryl-Carbonsäuren, die gegebenenfalls 1 bis 3 der zuvor genannten Substituenten enthalten können. Als Einzelbeispiele seien genannt: Ameisensäure, Essigsäure, Propionsäure, Buttersäuren, Valeriansäuren, Trimethylessigsäure, Fluor-, Difluor- und Trifluoressigsäure, Chlor-, Dichlor- und Trichloressigsäure, Chlorpropionsäure (α und β), Glykolsäure, Milchsäure, Cyanessigsäure, Oxalsäure, Bernsteinsäure, Malonsäure, Maleinsäure, Fumarsäure, Benzoesäure, Polyolsäuren, Chlorbenzoesäuren, Nitrobenzoesäuren, Hydroxybenzoesäuren, Phthalsäure, Isophthalsäure und Terephthalsäure.

Besonders bevorzugt sind Ameisen- und Oxalsäure, insbesondere Ameisensäure. Man kann auch Gemische von 2 oder mehr Carbonsäuren einsetzen. Die Säuren können wasserhaltig sein, vorzugsweise sind sie jedoch wasserfrei.

Das Molverhältnis der Carbonsäuren zum jeweiligen Dichlormethyl-2,2-difluorbenzo[1.3]dioxol kann beispielsweise im Bereich 1:1 bis 50:1 liegen. Vorzugsweise beträgt dieses Verhältnis 5:1 bis 30:1, insbesondere 10:1 bis 25:1. Man kann diese Umsetzung gegebenenfalls in Gegenwart von Wasser und/oder Katalysatoren durchführen. Als Katalysatoren kommen z.B. Metallchloride in Frage wie Aluminium(III)-, Bor(III)-, Eisen(III)-, Titan(IV)- und Zink(II)-chloride in Mengen bis zu 10 mol-%, bezogen auf das Edukt. Vorzugsweise arbeitet man ohne Katalysator. Die Umsetzung kann z.B. bei Temperaturen im Bereich von 0 bis 150°C durchgeführt werden. Bevorzugt sind Temperaturen im Bereich 20 bis 130°C, insbesondere 50 bis 110°C. Die Reihenfolge der Zugabe der Reaktionspartner ist beliebig. Wenn man bei erhöhter Temperatur arbeitet kann man die Reaktionspartner auch erst zusammenfügen und dann auf Reaktionstemperatur bringen. Wenn man mit Ameisensäure gearbeitet hat, kann man das nach der Reaktion vorliegende Gemisch z.B. destillativ, vorzugsweise bei vermindertem Druck, aufarbeiten und dabei zunächst überschüssige Ameisensäure und dann den jeweils hergestellten 2,2-Difluorbenzo[1.3]dioxol-carbaldehyd abtrennen. Wenn man mit Oxalsäure gearbeitet hat kann man das Reaktionsgemisch auf Wasser geben und aus der sich bildenden organischen Phase das Produkt durch Destillation gewinnen. Beim Einsatz anderer Säuren kann man analog verfahren.

Das erfindungsgemäße Verfahren hat eine Reihe von Vorteilen: So weist es 1 bis 2 Reaktionsstufen weniger auf als bekannte Verfahren. Dabei ist besonders überraschend, daß in der 1. Stufe des erfindungsgemäßen Verfahrens zwei sehr unterschiedliche Molekülteile chloriert werden können, nämlich die Methylengruppe in der Methylendioxybrücke und die Methylgruppe am Phenylring. Hinsichtlich Ausbeute, Chemikalieneinsatz und zu entsorgende Rückstände ist die Umsetzung eines Dichlormethyl-2,2-difluorbenzo[1.3]dioxols mit Carbonsäure wesentlich vorteilhafter als die bekannte Umsetzung mit Hexamethylentetramin/Essigsäure. Die schwierige Handhabung von Butyllithium ist nicht erforderlich. Es ist auch überraschend, daß die erfindungsgemäß durchzuführende Chlorierung mit elementaren Chlor ausführbar ist, da für entsprechende Chlorierungen des Standes der Technik nur Phosphorpentachlorid als Chlorierungsmittel genannt wird (siehe die eingangs beschriebenen Verfahren 1 und 3).

### Beispiele

Im folgenden gemachte Prozentangaben sind Gewichtsprozent, soweit nichts anderes vermerkt ist.

### Beispiel 1

### (Herstellung der Ausgangssubstanz 4-Methylbenzo[1.3]dioxol - nicht erfindungsgemäß)

Ein Gemisch aus 124 3-Methylbrenzkatechin, 255 g Dichlormethan, 233 g g Natriumcarbonat und 1000 ml Dimethylsulfoxid wurde für 22 Stunden bei 115°C gerührt. Dann wurden im Vakuum bei ca. 2 mbar und 120 bis 140°C alle flüchtigen Bestandteile abdestilliert und das Destillat auf 1000 ml Wasser gegeben. Die sich bildende organische Phase wurde abgetrennt und die wäßrige Phase mit Methyl-tert.-butylether extrahiert. Die vereinigten organischen Phasen wurden mit Wasser gewaschen, getrocknet und eingeengt. Man erhielt so 104,2 g (74,9 % der Theorie) 4-Methylbenzo[1.3]dioxol mit einer gaschromatografisch bestimmten Reinheit von 97,8 %.

Bei Verwendung von Kaliumcarbonat anstelle von Natriumcarbonat konnte die Ausbeute auf nahezu 90 % der Theorie gesteigert werden.

### Beispiel 2

### (Herstellung von 4-Dichlormethyl-2,2-dichlorbenzo[1.3]dioxol)

102 g 4-Methylbenzo[1.3]dioxol und 0,5 g AIBN wurden vorgelegt und unter Rühren auf 70°C erwärmt. Dann wurden innerhalb von 6 Stunden 114 g Chlor eingeleitet. Nun wurde die Ölbad-Temperatur auf 150°C erhöht und innerhalb von 18 Stunden weitere 114 g Chlor eingeleitet. Dann ließ man auf Raumtemperatur abkühlen. Die so erhaltenen 188 g des Produkt-Rohgemisches hatten folgende Zusammensetzung:
4 % 4-Methyl-2,2-dichlorbenzo[1.3]dioxol,
28 % 4-Chlormethyl-2,2-dichlorbenzo[1.3]dioxol,
49 % 4-Dichlormethyl-2,2-dichlorbenzo[1.3]dioxol und
5 % 4-Trichlormethyl-2,2-dichlorbenzo[1.3]dioxol.

Dieses Produktgemisch wurde über eine Kolonne destillativ getrennt. Das 4-Dichlormethyl-2,2-dichlorbenzo[1.3]dioxol ging dabei bei einem Siedepunkt von 99 bis 103°C bei 0,35 bis 0,45 mbar über. Das 4-Methyl- und das 4-Chlormethyl-2,2-dichlorbenzo[1.3]dioxol wurden ebenfalls isoliert und in den nächsten Ansatz der Chlorierung zurückgeführt.

### Beispiel 3

### (Herstellung von 4-Dichlormethyl-2,2-difluorbenzo[1.3]dioxol)

Zu 72 g wasserfreiem Fluorwasserstoff tropfte man bei -15°C 49,5 g 4-Dichlormethyl-2,2-dichlorbenzo[1.3]dioxol und rührte 3 Stunden bei -15°C nach. Dann wurde die Hauptmenge Fluorwasserstoff im Vakuum bei 50 mbar abgezogen. Nach Phasentrennung wurde die organische Phase mit Eiswasser geschüttelt, abgetrennt, getrocknet und destilliert. Man erhielt 43,3 g (97,5 % der Theorie) 4-Dichlormethyl-2,2-difluorbenzo[1.3]dioxol mit einer gaschromatografisch bestimmten Reinheit von 97,7 % und einem Siedepunkt von 97 bis 99°C bei 22 mbar.

### Beispiel 4

### (Herstellung von 2,2-Difluorbenzo[1.3]dioxol-4-carbaldehyd)

Ein Gemisch aus 48,2 g 97,3 %igem 4-Dichlormethyl-2,2-difluorbenzo[1.3]dioxol und 230 g Ameisensäure wurde auf 100°C erwärmt und 6 Stunden bei dieser Temperatur gerührt. Anschließend wurde zunächst überschüssige Ameisensäure im Vakuum abdestilliert und dann 28,6 g (= 78,1 % der Theorie) 2,2-Difluorbenzo[1.3]dioxol-4-carbaldehyd mit einem Siedepunkt von 70 bis 73°C bei 2,3 mbar und einer gaschromatografisch bestimmten Reinheit von 98,8 % aufgefangen.

## Patentansprüche

1. Verfahren zur Herstellung von 2,2-Difluorbenzo[1.3]dioxol-carbaldehyden der Formel (I) 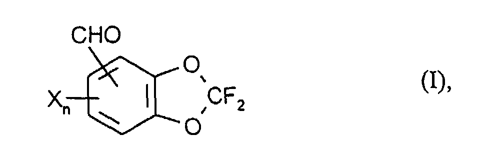 wobei
X für Fluor und/oder Chlor und
n für Null oder eine ganze Zahl von 1 bis 3 stehen,
dadurch gekennzeichnet, daß man Methylbenzo[1.3]dioxole der Formel 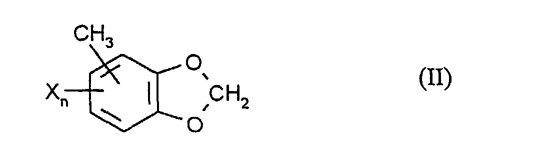 wobei X und n die bei Formel (I) angegebene Bedeutung haben,
chloriert, das erhaltene Dichlormethyl-2,2-dichlorbenzo[1.3]dioxol mit Fluorwasserstoff fluoriert und das dabei erhaltene Dichlormethyl-2,2-difluorbenzo[1.3]dioxol mit einer Carbonsäure umsetzt.

2. Verfahren nach Anspruch 1, dadurch gekennzeichnet, daß man als Chlorierungsmittel Chlor, Phosphorchloride, Sulfurylchlorid oder Gemische davon in Mengen von über 3,5 Chlorierungsäquivalenten pro Mol Methylbenzo[1.3]dioxol der Formel (II) einsetzt und bei Temperaturen im Bereich 0 bis 230°C arbeitet.

3. Verfahren nach Ansprüchen 1 und 2, dadurch gekennzeichnet, daß man die Chlorierung ohne Zusatz von Radikalstartern und in Abwesenheit von UV-Licht durchführt und bei Temperaturen von 60 bis 210°C arbeitet.

4. Verfahren nach Ansprüchen 1 und 2, dadurch gekennzeichnet, daß man die Chlorierung unter Zusatz von Radikalstartern oder in Gegenwart von UV-Licht durchführt und bei 40 bis 180°C arbeitet.

5. Verfahren nach Ansprüchen 1 bis 4, dadurch gekennzeichnet, daß man die Chlorierung bei Temperaturen von 40 bis 100°C beginnt und bei Temperaturen von 120 bis 180°C zu Ende führt.

6. Verfahren nach Ansprüchen 1 bis 5, dadurch gekennzeichnet, daß man in die Fluorierung mindestens 2 mol Fluorwasserstoff pro Mol Dichlormethyl-2,2-dichlorbenzo[1.3]dioxol einsetzt und bei Temperaturen im Bereich -35 bis +180°C arbeitet.

7. Verfahren nach Ansprüchen 1 bis 6, dadurch gekennzeichnet, daß man in die Umsetzung mit einer Carbonsäure 1:1 bis 50:1 Mole der Säure pro Mol Dichlormethyl-2,2-difluorbenzo[1.3]dioxol einsetzt.

8. Verfahren nach Ansprüchen 1 bis 7, dadurch gekennzeichnet, daß man die Umsetzung mit Ameisen- und/oder Oxalsäure bei Temperaturen im Bereich 0 bis 150°C durchführt.

## Claims

1. Process for the preparation of 2,2-difluorobenzo[1.3]dioxolecarbaldehydes of the formula (I) 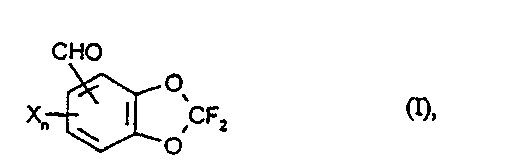 wherein
X represents fluorine and/or chlorine and
n represents zero or an integer from 1 to 3,
characterized in that methylbenzo[1.3]dioxoles of the formula 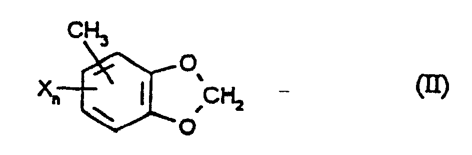 wherein X and n have the meaning given in the case of formula (I),
are chlorinated, the resulting dichloromethyl-2,2-dichlorobenzo[1.3]dioxole is fluorinated with hydrogen fluoride and the dichloromethyl-2,2-difluorobenzo[1.3]dioxole obtained by this procedure is reacted with a carboxylic acid.

2. Process according to Claim 1, characterized in that chlorine, phosphorus chlorides, sulphuryl chloride or mixtures thereof in amounts of more than 3.5 chlorination equivalents per mole of methylbenzo[1.3]dioxole of the formula (II) are employed as the chlorinating agent and the reaction is carried out at temperatures in the range from 0 to 230°C.

3. Process according to Claims 1 and 2, characterized in that the chlorination is carried out without the addition of free radical initiators and in the absence of UV light and the reaction is carried out at temperatures from 60 to 210°C.

4. Process according to Claims 1 and 2, characterized in that the chlorination is carried out with the addition of free radical initiators or in the presence of UV light and the reaction is carried out at 40 to 180°C.

5. Process according to Claims 1 to 4, characterized in that the chlorination is started at temperatures from 40 to 100°C and brought to completion at temperatures from 120 to 180°C.

6. Process according to Claims 1 to 5, characterized in that at least 2 mol of hydrogen fluoride per mole of dichloromethyl-2,2-dichlorobenzo[1.3]dioxole are employed in the fluorination and the reaction is carried out at temperatures in the range from -35 to +180°C.

7. Process according to Claims 1 to 6, characterized in that 1:1 to 50:1 moles of the acid per mole of dichloromethyl-2,2-difluorobenzo[1.3]dioxole are employed in the reaction with a carboxylic acid.

8. Process according to Claims 1 to 7, characterized in that the reaction is carried out with formic and/or oxalic acid at temperatures in the range from 0 to 150°C.

## Revendications

1. Procédé de préparation de 2,2-difluorobenzo-[1,3]dioxolcarbaldéhydes de formule (I) : 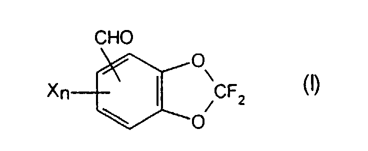 où
X représente fluor et/ou chlore, et
n représente zéro ou un nombre entier allant de 1 à 3,
caractérisé en ce que l'on réalise la chloration d'un méthylbenzo[1,3]dioxol de formule : 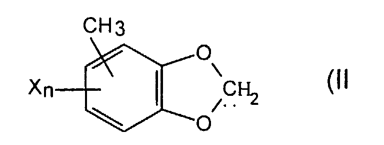 où X et n ont la signification donnée pour la formule (I), on réalise la fluoration du dichlorométhyl-2,2-dichlorobenzo[1,3]dioxol obtenu avec du fluorure d'hydrogène et on fait réagir le dichlorométhyl-2,2-difluorobenzo[1,3]dioxol ainsi obtenu avec un acide carboxylique.

2. Procédé suivant la revendication 1, caractérisé en ce que l'on met en oeuvre comme agent de chloration, du chlore, du chlorure de phosphore, du chlorure de sulfuryle, des mélanges de ceux-ci, en des quantités de jusqu'à 3,5 équivalents d'agent de chloration par mole de méthylbenzo[1,3]dioxol de formule (II) et en ce que l'on travaille à des températures dans l'intervalle allant de 0 à 230°C.

3. Procédé suivant les revendications 1 et 2, caractérisé en ce que l'on réalise la chloration sans addition d'initiateur radicalaire et en l'absence de lumière U.V. et en ce que l'on travaille à des températures allant de 60 à 210°C.

4. Procédé suivant les revendications 1 et 2, caractérisé en ce que l'on réalise la chloration avec addition d'un initiateur radicalaire ou en présence de lumière U.V. et que l'on travaille à 40-180°C.

5. Procédé suivant les revendications 1 à 4, caractérisé en ce que l'on commence la chloration à des températures allant de 40 à 100°C et en ce que l'on poursuit à des températures allant de 120 à 180°C.

6. Procédé suivant les revendications 1 à 5, caractérisé en ce que l'on met en oeuvre dans la fluoration, au moins 2 moles de fluorure d'hydrogène par mole de dichlorométhyl-2,2-dichlorobenzo[1,3]dioxol et en ce que l'on travaille à des températures dans l'intervalle allant de -35 à +180°C.

7. Procédé suivant les revendications 1 à 6, caractérisé en ce que l'on met en oeuvre, dans la réaction avec un acide carboxylique, de 1:1 à 50:1 moles d'acide par mole de dichlorométhyl-2,2-difluorobenzo[1,3]dioxol.

8. Procédé suivant les revendications 1 à 7, caractérisé en ce que l'on réalise la réaction avec de l'acide formique et/ou de l'acide oxalique à des températures dans l'intervalle allant de 0 à 150°C.
